# EUROPEAN PATENT SPECIFICATION

(11) **EP 1 586 566 B1**
(45) Date of publication and mention of the grant of the patent: **13.05.2009**
(21) Application number: 05006473.2
(22) Date of filing: 24.03.2005
(51) Int. Cl.: C07D 251/46

(54) **P-toluenosulfonate salt of N-Methyl-N-(3,5-dimathoxy-2,4,6-triazinyl-1-)-morpholine and related compounds for use as condensing reagent in peptide synthesis**
P-Toluenosulfonatsalz des N-Methyl-N-(3,5-dimatoxy-2,4,6-triazinyl-1-)-Morpholins und verwandte Verbindungen zur Verwendung als Kondensationsreagenz in der Peptidsynthese
Sél P-toluenosulfonate de la N-méthyle-N-(3,5-dimatoxy-2,4,6-triazinyl-1-)-morpholine et composés similaires pour l'utilisation comme réagents de condensation dans la synthèse de peptides

(30) Priority: 29.03.2004 PL 36667304
(43) Date of publication of application: 19.10.2005
(73) Proprietor: Politechnika Lodzka, 90-924 Lodz (PL); Kaminski, Zbigniew, 94-050 Lodz (PL); Kolesinska, Beata, 93-530 Lodz (PL)
(72) Inventor: Kaminski, Zbigniew, 94-050 Lodz (PL); Kolesinska, Beata, 93-530 Lodz (PL); Kolesinska, Justyna, 93-530 Lodz (PL); Jastrzabek, Konrad, 94-058 Lodz (PL)
(74) Representative: Balczewski, Zbigniew Wojciech

(56) References cited:
- WO-A-01/96282
- KUNISHIMA ET AL: "Approach to green chemistry of DMT-MM: recovery and recycle of coproduct to chloromethane-free DMT-MM" TETRAHEDRON LETTERS, vol. 43, no. 18, 2002, pages 3323-3326, XP002338627

## Description

The object-matter of the invention are new compounds, N-triazinylammonium salts and application of the new compounds.

Chlortriazynes and N-triazinylammonium salts of hydrochloric , fluoroboric, fluorophosphoric and perchloric acids are known among other from professional journals like Biopolymers, 55 (2) 140-165 and Australian Journal of Chemical Society, 54, 469 (2001) as well as from numerous patent specifications. The above mentioned triazyn compounds, as well as carbodiimides, uronium salts, guanidine salts and phosphonium derivatives of benzotriazole or of azobenzotriazole are currently the most often used condensing reagents in production of nucleic acids, peptides, amides, esters and carboxylic acid anhydrides, in reactions of hydrogenation carboxylic functional groups to aldehyde groups as well as reagents used for protection of functional groups. However, they have some inconveniences. Namely, chlorotriazines and triazinylammonium chlorides are susceptible to dealkilation in presence of strong nucleophilic chloride anion. Triazinylammonium salts of fluoroboric and fluorophosphoric acids pollute the environment. Carbodiimides are susceptible to migration of the acyl group O->N, develop allergic reactions and processes in which they are involved are often low effective. The uronium, guanidinium and phosphonium salts are hardly available due to high production costs, with exception of uronium salts ofN-oxide of 2-mercapto-pyridone which are more easily available but their application is limited due to presence of strong nucleophilic sulfur atom, which develops many side reactions.

The invention refers to quarternary N-(3,5-disubstituted -1,3,5-triazinyl-1)ammonium salts of sulfonic acids, of the formula 1, 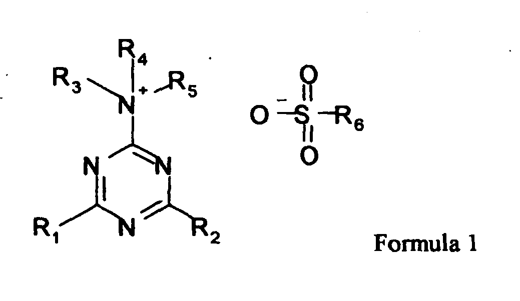 where R₁ and R₂ denote independently *of* each other a halogen atom, an alkyl group, a substituted alkyl group, an alkoxy group, a substituted alkoxy group, a cycloalkoxy group, a substituted cycloalkoxy group, an aryl group, a substituted aryl group, an aryloxy group, a substituted aryloxy group, a heterocyclic group or a substituted heterocyclic group, R₃, R₄, R₅ denote independently *of* each other an alkyl group, a substituted alkyl group, a cycloalkyl group, a substituted cycloalkyl group, an aryl group, a substituted aryl group, a heterocyclic group or a substituted heterocyclic group, or form *an unsubstituted* heterocyclic ring *together* with *the* nitrogen atom, whereas ⁻O-SO₂R₆ denotes *a benzenosulfonate anion, a p-toluenosulfonate anion, a p-bromobenzenosulfonate anion, a p-chlorobenzenosulfonate anion, a methanosulfonate anion* or an amidosulfonate anion. The R₁ and R₂ denote the methoxy group.

The new compounds of the formula 1 are designed as condensing reagents in the synthesis of amides, esters, carboxylic acid anhydrides, peptides and peptide esters.

The new compounds of the formula 1 are formed in reaction of sulfonic acids *salts* of the formula 2, 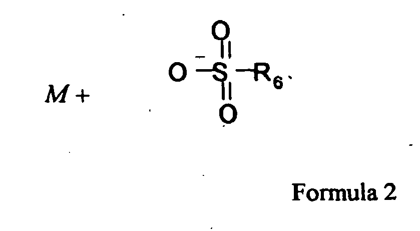 where ⁻O-SO₂R₆ denotes the above specified groups, preferable of lithium or silver salts, with quarternary triazinylammonium chlorides or by simultaneous reactions of sulfonic acids *salts of the* formula 2, with 2-chloro-4,6-disubstituted-1,3,5-triazine, preferable with 2-chloro-4,6-dimethoxy-1,3,5-triazine and tertiary amines.

The new compounds are more easily available and less expensive as compared to the known triazinylammonium salts, and their anions (sulfonic) are not toxic and readily biodegradable, therefore safe for the natural environment. Moreover, these compounds are considerable more resistant to dealkylation, than other known triazinylammonium salts, providing very good results of condensation, as compared to other commonly used condensating reagents.

The object-matter of the invention is illustrated by following examples, which will not limit of its range.

### Example I.

Mixture of 17.8 g (100 mmol) of lithium p-toluenosulfonate and 27.6 g (100 mmol) of N-methyl-N-(3,5-dimethoxy -2,4,6-triazinylo-1)morpholine chloride in 100 ml acetonitrile was cooled down to a temperature approximating to 5°C and intensively stirred for 3 hours, next the precipitate was separated by filtration. The precipitate was two times washed with acetonitrile and the collected filtrates were evaporated to dryness under reduced pressure. Dry residue was washed with 50 ml tetrahydrofurane and crystallized from acetonitrile. It was obtained 31.7 g p-toluenosulfonate of N-methyl-N-(3,5-dimethoxy-2,4,6--triazinyl-1-)-morpholine, what equals to 77% yield. The new compound was characterized with the following data:
melting point = 59 - 60 °C,
Results of elementary analysis of the formula : C₁₇H₂₄N₄O₆S (412.47)

| | | | | |
|---|---|---|---|---|
| calculated: | %C 49.50, | %H 5.87, | %N 13.58, | %S 7.77 |
| found: | %C 48.67, | %H 5.54, | %N 13.61, | %S 7.63. |

### Example II.

Mixture of 17.8 g (100 mmol) of lithium p-toluenosulfonate and 100 ml acetonitrile was cooled down to a temperature approximating to 0°C and, intensively stirring, simultaneously added dropwise 50 ml of solution of 17.6 g (100 mmol) 2-chloro-4,6-dimethoxy-1,3,5-triazine in acetonitrile and 11 ml (100 mmol) of N-methylomorpholine. The stirring of the mixture was continued at a temperature of 0°C for 2 hours, next the precipitate was separated by filtration. The precipitate was washed with 3 portions by 30 ml of acetonitrile and the collected filtrates were evaporated to dryness. The residue was washed with 25 ml tetrahydrofurane and crystallized from acetonitrile.

It was obtained 30.6 g p-toluenosulfonate of N-methyl-N-(3,5-dimethoxy-2,4,6-triazinyl-1-)--morpholine, what equals to 74% yield. The new compound was characterized with the following data:
melting point = 59 - 60 °C,
Results of elementary analysis of the formula: C₁₇H₂₄N₄O₆S

| | | | | |
|---|---|---|---|---|
| calculated: | %C 49.50, | %H 5.87, | %N 13.58, | %S 7.77 |
| found: | %C 48.79, | %H 5.48, | %N 13.49, | %S 7.55. |

### Example III.

23,8 g (100 mmol) of lithium 10-camphorosulfonate and 80 ml acetonitrile was cooled down to a temperature approximating to 5°C and, intensively stirring, simultaneously added dropwise the solution of 17.6 g (100 mmol) of 2-chloro-4,6-dimethoxy-1,3,5-triazine in 50 ml acetonitrile and simultaneously 11 ml (100 mmol) N-methylmorpholine. The stirring of the mixture was continued at a temperature of 0°C for 2 hours, next the precipitate was separated by filtration. The precipitate was washed with acetonitrile (3 x 30 ml) and the collected filtrates were evaporated to dryness. The residue was washed with tetrahydrofurane (25 ml) and crystallized from acetonitrile.
It was obtained 28.4 g of 10-camphorosulfonate of N-methyl-N-(3,5-dimethoxy-2,4,6-triazinyl-1-)-morpholine, what equals to 60% yield.
The new compound was characterized with the following data:
Melting point =127-129°C,
Results of elementary analysis of the formula: C₂₀H₃₂N₄O₇S (472,56)

| | | | | |
|---|---|---|---|---|
| calculated: | %C 50.83, | % H 6.83, | % N 11.86, | % S 6.79 |
| found: | % C 48.84, | % H 6.83 | % N 13.18, | % S 5.48. |

### Example IV.

To a mixture of 17.8 g (100 mmol) of lithium p-toluenosulfonate in 30 ml acetonitrile, cooled down to a temperature approximating to 0°C, simultaneously added drop by drop solution of 1.76 g (10 mmol) CDMT in 20 ml of acetonitrile and solution of 1.11 g (10 mmol) of quinuclidine in 5 ml acetonitrile. Then, the stirring of the mixture was continued at a temperature of 0°C for 2 hours, next the precipitate was separated by filtration. The precipitate was washed with 2 portions by 25 ml acetonitrile and the collected filtrates were evaporated to dryness. The residue was washed with ethyl ether and crystallized from acetonitrile.
It was obtained 2.95 g of p-toluensulfonate of N-(3,5-dimethoxy-2,4,6-triazinyl-1-)-quinuclidine in the form of thick oily liquid, what equals to 70% yield. The new compound was characterized with the following data:
Results of elementary analysis of the formula: C₁₉H₂₆N₄O₅S (422.51)

| | | | | |
|---|---|---|---|---|
| calculated: | %C 54.01; | %H 6.20; | %N 13.26; | %S 7.59 |
| found: | %C 54.04; | %H 6.16, | %N 13.38, | %S 7.22. |

### Example V.

To an intensively stirred mixture of 1.02 g (10 mmol) of lithium methansulfonate in 30 ml acetonitrile, cooled down to a temperature approximating to 0°C, simultaneously added drop by drop a solution of 1.75 g (10 mmol) CDMT in acetonitrile and solution of 1.1 g (10 mmol) of N-methylmorpholine in 5 ml acetonitrile. Then, the stirring of the mixture was continued at a temperature of 0°C for 2 hours, next the precipitate was separated by filtration. The precipitate was washed with 2 portions by 25 ml acetonitrile and the collected filtrates were evaporated to dryness. The residue was washed with ethyl ether, dried and crystallized from acetonitrile.
It was obtained 0.185 g methansulfonate of N-methyl-N-(3,5-dimethoxy-2,4,6-triazinyl-1-)--morpholine, what equals to 55 % yield. The new compound was characterized with the following data:
Melting point: 96 - 98°C
Results of elementary analysis of the formula C₁₁H₂₀N₄O₆S (336,37):

| | | | | |
|---|---|---|---|---|
| calculated: | %C 39.28 | %H 5,99 | %N 16.66 | %S 9.53, |
| found: | %C 37.45 | %H 6.14 | %N 16.20 | %S 8.61. |

### Example VI.

0,4125 g (1 mmol) p-toluenesulfonate of N-methyl-N-(3,5-dimethoxy-2,4,6-triazinyl-1)-morpholine was dissolved in 5 ml acetonitrile and added 0.3294 g (1mmol) Fmoc-AlaOH, next the mixture was cooled down in an ice/water bath and added 0.110 ml (1 mmol) of NMM. After 2 hours, to the intensively cooled and stirred mixture 0.1817 g (1 mmol) H-Leu-OMe x HCl and 0.110 ml (1 mmol) NMM was added. The mixture was left for 12 hours at room temperature, then acetonitrile was removed in a vacuum evaporator. The residue was dissolved in 5 ml of ethyl acetate and washed one by one with water, NaHSO₄, water, NaHCO₃ and again with water, the organic layer was dried over MgSO₄, then the drying agent was removed by filtration and the solvent was evaporated to dryness.
It was obtained 0.3815 g of Fmoc-Ala-Leu-OMe dipeptide, what equals to 87% yield. The NMR analysis gave following results:
¹H-NMR (CDCl₃):δ = 0,88 (d, 6H, J = 15 Hz, (CH₃)₂-CH-), 1,30 (d, 3H, J=12 Hz, CH₃CH-), 1,48-1,67 (m, 2H, (CH₃)₂CHCH₂-), 3,63 (s, 3H, CH₃O-), 4,02 (t, 1H, J=6 Hz, -CHCH₂O-), 4,18 (d, 2H, J=9,5 Hz, -CHCH₂O), 4,32 (q, 1H, J=5 Hz, (CH₃)₂CHCH₂-), 4,50 (qu, 1H, CH₃CH-), 7,08-7,66 (8H, arom) [ppm].

### Example VII.

0,423 g (1 mmol) p-toluenesulfonate of N-(3,5-dimdthoxy-2,4,6-triazinyl-1-)-quinuclidine was dissolved in 5 ml acetonitrile and added 0.3294 g (1mmol) Fmoc-Ala-OH, next the mixture was cooled down in an ice/water bath and added 0.110 ml (1 mmol) of NMM. After 2 hours, to the intensively cooled and stirred mixture 0.1396 g (1 mmol) H-Ala-OMe x HCl and 0.110 ml (1 mmol) NMM was added. The mixture was left for 12 hours at room temperature, and then acetonitrile was removed in a vacuum evaporator. The residue was dissolved in 5 ml of ethyl acetate and washed one by one with water, NaHSO₄, water, NaHCO₃ and again with water. The organic layer was dried over MgSO₄, then the drying agent was removed by filtration and the solvent was evaporated to dryness.
It was obtained 0.3608 g of Fmoc-Ala-Ala-OMe dipeptide, what equals to 91% yield. The NMR analysis of the dipeptide gave following results:
¹H-NMR (CDCl₃):δ = 1,23 (d, 3H, J = 12 Hz, CH₃-CH-), 1,32 (d, 3H, J=12 Hz, CH₃CH-), 3,61 (s, 3H, CH₃O-), 3,99-4,04 (m, 2H, -CHCH₂O- i -COCHCH₃), 4,18 (d, 2H, J=9,5 Hz, -CHCH₂O), 4,49 (qu, 1H, CH₃CH-), 7,08-7,72 (8H, arom) [ppm].

### Example VIII.

0,4125 g (1 mmole) p-toluenesulfonate of N-methyl-N-(3,5-dimethoxy-2,4,6-triazinyl-1-)--morpholine was dissolved in 5 ml acetonitrile and added 0.3294 g (1mmol) Fmoc-Ala-OH, next the mixture was cooled down in an ice/water bath and added 0.110 ml (1 mmol) of NMM. After 2 hours, to the intensively cooled and stirred mixture 0.2157 g (1 mmole) H-Phe-OMe x HCl and 0.110 ml (1 mmole) NMM was added. The mixture was left for 12 hours at room temperature, then acetonitrile was removed in a vacuum evaporator. The residue was dissolved in 5 ml of ethyl acetate and washed one by one with water, NaHSO₄, water, NaHCO₃ and again with water. The organic layer was dried over MgSO₄, then the drying agent was removed by filtration and the solvent was evaporated to dryness. It was obtained 0.4158 g of Fmoc-Ala-Phe-OMe dipeptide, what equals to 88% yield. The NMR analysis of the dipeptide gave following results:
¹H-NMR (CDCl₃):δ = 1,30 (d, 3H, J = 12 Hz, CH₃-CH-), 3,06-3,08 (m, 2H, -CH₂-C₆H₅), 3,55 (s, 3H, CH₃O-), 4,04 (t, 1H, J= 6,5 Hz, -CHCH₂O-), 4,18 (d, 2H, J=9,5 Hz, -CHCH₂O), 4,47 (qu, 1H, J=6,5 Hz, CH₃CH-), 4,86 (q, 1H, J=7 Hz, -CH-CH₂-), 7,08-7,72 (13H, arom) [ppm].

### Example IX.

0,4125 g (1 mmol) p-toluenesulfonate of N-methyl-N-(3,5-dimethoxy-2,4,6-triazinyl-1-)--morpholine was dissolved in 5 ml acetonitrile and added 0.2373 g (1mmol) Z-Aib-OH, next the mixture was cooled down in an ice/water bath and added 0.110 ml (1 mmol) ofNMM. After 2 hours, to the intensively cooled and stirred mixture 0.1396 g (1 mmol) H-Ala-OMe x HCl and 0.110 ml (1 mmol) NMM was added. The mixture was left for 12 hours at room temperature. Then, acetonitrile was removed in a vacuum evaporator. The residue was dissolved in 5 ml of ethyl acetate and washed one by one with water, NaHSO₄, water, NaHCO₃ and again with water. The organic layer was dried over MgSO₄, then the drying agent was removed by filtration and the solvent was evaporated to dryness.
It was obtained 0.2741 g of Z-Aib-Ala-OMe dipeptide, what equals to 85% yield.
¹H-NMR (CDCl₃):δ = 1,33 (d, 3H, J = 10 Hz, CH₃-CH-), 1,49 (s, 6H, (CH₃)₂C-), 3,62 (s, 3H, CH₃O-), 4,46 (qu, 1H, CH₃CH-), 5,06 (AB system, 2H, J= 8,5 Hz, -CH₂O-), 7,28-7,36 (5H, arom) [ppm].

### Example X.

0.473 g (1 mmol) of N-methyl-N-(3,5-dimethoxy-2,4,6-triazinyl-1-)-morpholine 10--camphorsulfonate was dissolved in 5 ml acetonitrile, 0.2373 g (1 mmol) Z-Aib-OH was added, then the reaction mixture was cooled down on an ice/water bath and 0.110 ml (1 mmole) NMM was added. After 2 hours, to the intensively cooled and stirred mixture 0.2157 g (1 mmole) H-Phe-OMe XHCl and 0.110 ml (1 mmole) NMM were added. The mixture was left at room temperature for 12 hours. Next, acetonitrile was removed in a vacuum evaporator, and the residue was dissolved in 5 ml of ethyl acetate and washed successively with water, NaHSO₄, water, NaHCO₃ and again with water. The organic layer was dried over MgSO₄, then the drying agent was removed by filtration and the solvent was evaporated to dryness.
It was obtained 0.3227 g of Z-Aib-Phe-OMe dipeptide, what equals to 81% yield.
The NMR analysis of the dipeptide gave following results:
¹H-NMR (CDCl₃):δ = 1,50 (s, 6H, (CH₃)₂C-), 3,08-3,10 (m, 2H, -CH₂CH-), 3,59 (s, 3H, CH₃O-), 4,85-4,89 (m, 1H, -CH₂CH-), 5,06 (AB system, 2H, J = 8,5 Hz, -CH₂O-), 7,26-7,37 (10H, arom) [ppm].

### Example XI.

0,4125 g (1 mmol) p-toluenesulfonate of N-methyl-N-(3,5-dimethoxy-2,4,6-triazinyl-1-)--morpholine was dissolved in 5 ml acetonitrile and added 0.2373 g (1mmol) Z-Aib-OH, next the mixture was cooled down in an ice/water bath and added 0.110 ml (1 mmol) of NMM After 2 hours, to the intensively cooled and stirred mixture 0.1817 g (1 mmol) H-Leu-OMe x HCl and 0.110 ml (1 mmol) NMM was added. The mixture was left for 12 hours at room temperature. Then, acetonitrile was removed in a vacuum evaporator. The residue was dissolved in 5 ml of ethyl acetate and washed one by one with water, NaHSO₄, water, NaHCO₃ and again with water. The organic layer was dried over MgSO₄, then the drying agent was removed by filtration and the solvent was evaporated to dryness.
It was obtained 0.3062 g of Z-Aib-Leu-OMe dipeptide, what equals to 84% yield.
¹H-NMR (CDCl₃):δ = 0,86 (d, 6H, J = 8,5 Hz, (CH₃)₂-CH-), 1,46-1,68 (m, 2H, -CH₂CH-), 1,50 (s, 6H, (CH₃)₂C-), 3,63 (s, 3H, CH₃O-), 4,28 (q, 1H, -CH₂CH-), 5,06 (AB system, 2H, J = 8,5 Hz, -CH₂O-), 7,29-7,34 (5H, arom) [ppm].

### Example XII.

0,4125 g (1 mmol) p-toluenesulfonate of N-methyl-N-(3,5-dimethoxy-2,4,6-triazinyl-1-)-morpholine was dissolved in 5 ml acetonitrile and added 0.3664 g (1mmole) Boc-Orn(Z) -OH, next the mixture was cooled down in an ice/water bath and added 0.110 ml (1 mmole) of NMM After 2 hours, to the intensively cooled and stirred mixture 0. 1817 g (1 mmole) H-Leu-OMe x HCl and 0.110 ml (1 mmol) NMM was added. The mixture was left for 12 hours at room temperature. Then, acetonitrile was removed in a vacuum evaporator. The residue was dissolved in 5 ml of ethyl acetate and washed one by one with water, NaHSO₄, water, NaHCO₃ and again with water. The organic layer was dried over MgSO₄, then the drying agent was removed by filtration and the solvent was evaporated to dryness.
It was obtained 0.4393 g of Boc-Orn(Z)-Leu-OMe dipeptide, what equals to 89% yield. The NMR analysis of the dipeptide gave following results:
¹H-NMR (CDCl₃):δ = 0,86 (d, 6H, J = 8,5 Hz, (CH₃)₂-CH-), 1,41-1,70 (m, 6H, -CH₂CH- and -CH2CH₂CH₂CH-), 1,47 (s, 9H, (CH₃)₃C-), 3,37 (q, 2H, J= 4,5 Hz, -NH-CH₂CH₂CH₂CH-), 3,63 (s, 3H, CH₃O-), 3,93 (q, 1H, J=4,5 Hz, -CH₂-CH-CONH-), 4,29 (q, 1H, J=4,0 Hz, -CONH-CH-CH₂-), 5,06 (AB system, 2H, J = 8,5 Hz, -CH₂O-), 7,35 (s, 5H, arom) [ppm].

### Example XIII

0,4125 g (1 mmol) p-toluenesulfonate of N-methyl-N-(3,5-dimethoxy-2,4,6-triazinyl-1-)-morpholine was dissolved in 5 ml acetonitrile and added 0.823 g (2 mmol) Fmoc-Asp(O^{t}Bu)-OH, next the mixture was cooled down in an ice/water bath and added 0.220 ml (2 mmol) of NMM. After 2 hours, to the intensively cooled and stirred mixture 0,386 g, (2 mmol) pentaflurophenol and 0.220 ml (2 mmol) NMM was added. The mixture was left for 12 hours at room temperature. Then, acetonitrile was removed in a vacuum evaporator. The residue was dissolved in 10 ml of ethyl acetate and washed one by one with water, NaHSO₄, water, NaHCO₃ and again with water. The organic layer was dried over MgSO₄, then the drying agent was removed by filtration and the solvent was evaporated to dryness. It was obtained 1.016 g of pentafluorophenyl ester (Fmoc-Asp(O^{t}Bu)-OPfp), what equals to 88% yield.
The NMR analysis of the dipeptide gave following results:
¹H-NMR (CDCl₃):δ = 1,43 (s, 9H, (CH₃)₃CO-), 2,96 (ukl AB, 2H, J₁ = 8 Hz, J₂ = 12 Hz -OCOCH₂CH-), 4,20-4,60 (m, 3H, -CHCH₂OCONH-), 4,95 (dt, 1H, J=12 Hz, -OCOCH₂CH-), 7,08=7,76 (m, 8H, arom.) [ppm].

### Example XIV.

0.825 g (2 mmol) of p-toluenosulfonate of N-methylo-N-(3,5-dimethoxy-2,4,6-triazinyl-1-) -morpholine, was dissolved in 10 ml acetonitrile, next 0.823 g (2 mmol) Fmoc-Asp(O^{t}Bu)--OH were added. Then, the mixture was cooled down on the ice/water bath and 0.220 ml (2 mmol) NMM were added. After 2 hours, to the intensively cooled an stirred suspension 0.2 ml (3 mmol) of allyl alcohol were added and the mixture was left at room temperature for 12 hours. Next, acetonitrile was removed in a vacuum evaporator, the residue was dissolved in 10 ml ethyl acetate and washed one by one with water, NaHSO₄, water, NaHCO₃ and again with water. The organic layer was dried over MgSO₄, then the drying agent was removed by filtration and the solvent was evaporated to dryness.
It was obtained 0.777 g of allyl ester (Fmoc-Asp(O^{t}Bu)-OAll), what equals to 86% yield. The NMR analysis of the ester gave following results:
¹H-NMR (CDCl₃):δ = 1,43 (s, 9H, (CH₃)₃CO-), 2,86 (ukl AB, 2H, J₁ = 7 Hz, J₂ = 10 Hz -OCOCH₂CH-), 4,08-4,39 (m, 3H, -CHCH₂OCONH-), 4,65 (dt, 1H, J=12 Hz, -OCOCH₂CH-), 4,71 (d, 2H, J = 6 Hz, -OCH2CHCH2), 5,27-5,35 (m, 2H, -OCH2CHCH2), 5,58-5,62 (m, 1H, -OCH2CHCH2), 7,08-7,75 (m, 8H, arom) [ppm].

### Example XV.

0,4125 g (1 mmol) p-toluenesulfonate of N-methyl-N-(3,5-dimethoxy-2,4,6-triazinyl-1-) morpholine was dissolved in 5 ml acetonitrile and added 0.346 g (1 mmole) Fmoc-Ala-OH, next the mixture was cooled down in an ice/water bath and added 0.110 ml (12 mmole) of NMM. After 2 hours, to the intensively cooled and stirred mixture 0,156 g, (1 mmole) natural menthol was added. The mixture was left for 12 hours at room temperature. Then, acetonitrile was removed in a vacuum evaporator. The residue was dissolved in 10 ml of ethyl acetate and washed one by one with water, NaHSO₄, water, NaHCO₃ and again with water. The organic layer was dried over MgSO₄, then the drying agent was removed by filtration and the solvent was evaporated to dryness.
It was obtained 0.387 g of menthyl ester (Fmoc-Ala-O-menthol), what equals to 86% yield. The NMR analysis of the ester gave following results:
¹H-NMR (CDCl₃):δ = 0,81 (d, 3H, J = 12 Hz, CH₃-CH-menthol), 1,23 (d, 6H, J=12 Hz, (CH₃)₂CH-), 1,42 (d, 3H, J = 10 Hz, CH₃CH-), 1,06-2,09 (m, 8H, menthol), 3,75 (dq, 1H, O-CH-menthol), 4,35 (t, 1H, J=6 Hz, -CHCH₂O-), 4,65 (d, 2H, J=9,5 Hz, -CHCH₂O), 4,75 (qu, 1H, CH₃CH-), 7,08-7,66 (8H, arom) [ppm].

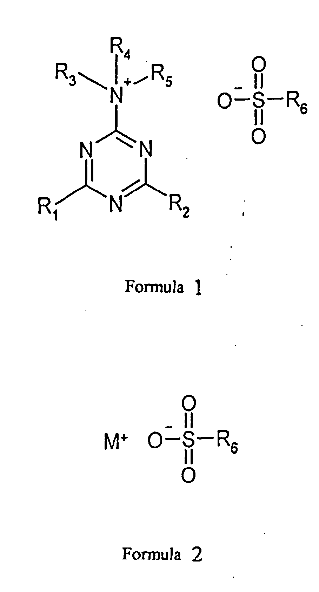

## Claims

1. Quaternary N-(3,5-disubstituted-1,3,5-tiazinyl-1)-ammonium salts of sulfonic acids of the formula 1, 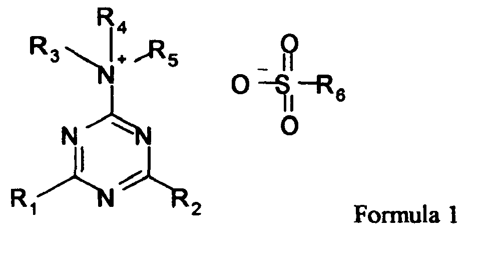 where R₁ and R₂ denote independently of each other a halogen atom, an alkyl group, a substituted alkyl group, an alkoxy group, a substituted alkoxy group, a cycloalkoxy group, a substituted cycloalkoxy group, an aryl group, a substituted aryl group, an aryloxy group, a substituted aryloxy group, a heterocyclic group or a substituted heterocyclic group, R₃, R₄, R₅ denote independently of each other an alkyl group, a substituted alkyl group, a cycloalkyl group, a substituted cycloalkyl group, an aryl group, a substituted aryl group, a heterocyclic group or a substituted heterocyclic group or form an unsubstituted heterocyclic ring together with *the* nitrogen atom, whereas ⁻O-SO₂R₆ denotes a *benzenosulfonate anion, a p-toluenosulfonate anion, a p-bromobenzenosulfonate anion, a p-chlorobenzenosulfonate, anion, a methanosulfonate anion* or an amidosulfonate anion.

2. The salts of the formula 1 as claimed in claim 1 **characterized in that** the R₁ and R₂ denote the methoxy group.

3. Use of the compounds of claim 1 as condensing reagents in the synthesis of amides, esters, carboxylic acid anhydrides, peptides and their esters.

## Patentansprüche

1. Quaternäre Ammoniumsalzen N-(3,5-disubstituiert-1,3,5-triazinyl-1)- der Sulfonsäuren aus der Formel 1, 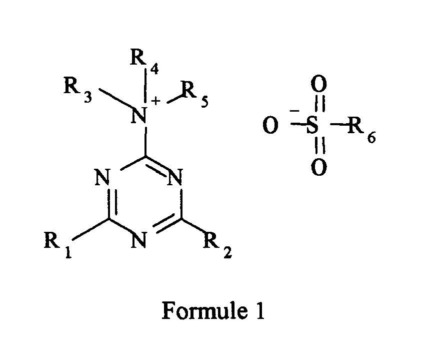 wo R₁ und R₂ unabhängig von einander ein Halogenatom, eine Alkylgruppe, eine substituierte Alkylgruppe, eine Alkoxylgruppe, eine substituierte Alkoxylgruppe, eine Cycloalkoxyl-Gruppe, eine substituierte Cycloalkoxyl-Gruppe, eine Arylgruppe, eine substituierte Arylgruppe, eine Aryloxygruppe, eine substituierte Aryloxygruppe, eine heterozyklische Gruppe oder eine substituierte heterozyklische Gruppe bezeichnen wo R₂, R₄, R₅ unabhängig von einander eine Alkylgruppe, eine substituierte Alkylgruppe, eine Cycloalkyl-Gruppe, eine substituierte Cycloalkyl-Gruppe, eine Arylgruppe, eine substituierte Arylgruppe, eine heterozyklische Gruppe oder eine substituierte heterozyklische Gruppe bezeichnen oder einen unsubstituierten heterozyklischen Ring *zusammen* mit dem Stickstoffatom bilden, wobei O-SO₂R₆ einen Benzolsulfonat-, p-toluensulfonat-, p-Bromobenzolsulfonat-, Chlorobenzolsulfonat-, Methansulfonat-Anion oder einen Amidosulfonat-Anion bezeichnet.

2. Die Salzen mit der Formel 1, wie in Anspruch 1, charakterisieren sich dadurch, dass R₁ i R₂ die Metoxylgruppe bezeichnen.

3. Die Benutzung der Verbindungen aus dem Anspruch 1 als kondensierende Reagens in der Synthese von Amiden, Ester, Carbonsäure-Anhydriden, Peptidbindungen und deren Ester.

## Revendications

1. Les seuls quaternaires N-(3,5 disubstitués-1,3,5-triazinylo-1)-ammonium des acides sulfoniques de la formule 1, 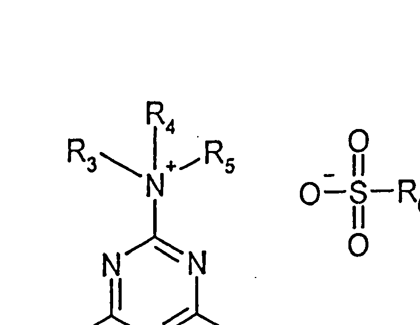 où R₁ et R₂ désignent indépendamment un atome d'halogène, un groupe alcoylique, un groupe alcoylique substitué, un groupe cycloalcoxilique, un groupe cycloalcoxilique substitué, un groupe arylique, un groupe arylique substitué, un groupe aryloxilique, un groupe aryloxilique substitué, un groupe hétérocyclique ou un groupe hétérocyclique substitué, R₂, R₄, R₅ désignent indépendamment un groupe cycloalcoylique, un groupe cycloalcoylique substitué, un groupe arylique, un groupe arylique substitué, un groupe hétérocyclique ou un groupe hétérocyclique substitué ou ils forment un noyau hétérocyclique non substitué avec des atomes de l'azote, pendant que O-SO₂R₆ désigne un anion benzenosulfonate, p-tolouenosulfonate, p-bromobenzenosulfonate, chlorobenzenosulfonate, méthanosulfonate ou amidosulfonate.

2. Les seuls ayant la formule 1, comme constatés à la revendication, se caractérisent par la présence du groupe méthoxylique dans les positions R₁ et R₂.

3. L'utilisation des composés de la revendication 1 comme des réactifs condensant dans la synthèse des amides, des esters, des anhidrites de l'acide carboxylique, des protéines et de leurs esters.
